# EUROPÄISCHE PATENTSCHRIFT

(11) **EP 2 621 330 B1**
(45) Veröffentlichungstag und Bekanntmachung des Hinweises auf die Patenterteilung: **05.04.2017**
(21) Anmeldenummer: 11764503.6
(22) Anmeldetag: 15.09.2011
(51) Int. Cl.: G01B 11/00, G06K 9/00, A61B 3/11, A61B 3/113, G01B 9/02, A61B 3/10

(54) **VERFAHREN UND VORRICHTUNG ZUR INTERFEROMETRISCHEN BESTIMMUNG VERSCHIEDENER BIOMETRISCHER PARAMETER EINES AUGES**
METHOD AND DEVICE FOR DETERMINING VARIOUS BIOMETRIC PARAMETERS OF AN EYE BY INTERFEROMETRY
PROCÉDÉ ET DISPOSITIF POUR LA DÉTERMINATION INTERFÉROMÉTRIQUE DE DIFFÉRENTS PARAMÈTRES BIOMÉTRIQUES D'UN IL

(30) Priorität: 29.09.2010 DE 102010047053
(43) Veröffentlichungstag der Anmeldung: 07.08.2013
(73) Patentinhaber: Carl Zeiss Meditec AG, 07745 Jena (DE)
(72) Erfinder: SCHMITT-MANDERBACH, Tobias, 07745 Jena (DE); BUBLITZ, Daniel, 07646 Rausdorf (DE); BERGNER, Roland, 07745 Jena (DE)
(74) Vertreter: Beck, Bernard
(86) Internationale Anmeldenummer: PCT/EP2011/065999
(87) Internationale Veröffentlichungsnummer: WO 2012/041712

(56) Entgegenhaltungen:
- WO-A2-01/01064
- WO-A2-02/065899
- WO-A2-03/003909
- WO-A2-2004/103169
- DE-A1- 10 349 230
- DE-A1- 19 857 001
- US-A- 5 889 577
- US-B1- 6 779 891
- DREXLER W ET AL: "SUBMICROMETER PRECISION BIOMETRY OF THR ANTERIOR SEGMENT OF THE HUMAN EYE", INVESTIGATIVE OPHTHALMOLOGY & VISUAL SCIENCE, ASSOCIATION FOR RESEARCH IN VISION AND OPHTHALMOLOGY, US, Bd. 38, Nr. 7, 1. Juni 1997 (1997-06-01), Seiten 1304-1313, XP009035980, ISSN: 0146-0404 in der Anmeldung erwähnt

## Beschreibung

Die vorliegende Erfindung betrifft eine Lösung zur interferometrischen Bestimmung verschiedener biometrischer Parameter eines Auges, bei der die optische Achse des biometrischen Messsystems zur optischen Achse eines Auges ausgerichtet wird und idealer Weise mit dieser zusammenfällt.

Während die optische Achse des Auges durch die Gerade zwischen den Krümmungsmittelpunkten von brechenden Flächen gekennzeichnet ist, wird als Sehachse, die Achse bezeichnet, die von der "Fovea centralis" durch den Knotenpunkt des Auges zum Fixierobjekt verläuft. Werden die verschiedenen Medien auf ein einziges Medium mit mittlerem Brechungsvermögen und kugelförmiger Krümmung durch Rechnung reduziert, so kann man einen Punkt im Auge angeben, durch welchen alle Strahlen ungebrochen durchgehen. Diesen Punkt bezeichnet man als Knotenpunkt der Sehachsen.

Die Sehachse weicht in der Regel bei allen Augen von der optischen Achse ab. Dies resultiert zum einen aus Abbildungsfehlern des Auges, die beispielsweise daraus resultieren, dass Krümmungsradien der einzelnen Augenmedien nicht gleichmäßig sind, die Augenlinse verkippt ist, die Retina sich nicht im Fokus der Augenlinse befindet und vieles mehr. Zum anderen wird beim Ausrichten des Auges auf ein Objekt versucht, dieses möglichst in der Fovea, als dem Bereich des schärfsten Sehens abzubilden.

Während die Ausrichtung des Auges für viele Untersuchungen in der Augenheilkunde keine Rolle spielt, ist zumindest die Kenntnis dessen Ausrichtung zum ophthalmologischen Gerät nicht nur für dessen Behandlung sondern auch für dessen Vermessung zwingend erforderlich.

Die Bestimmung verschiedener biometrischer Parameter eines Auges ist insbesondere vor einem operativen Eingriff zum Austausch der Augenlinse bei Vorliegen einer Linsentrübung (Katarakt) erforderlich. Um ein optimales Sehvermögen nach der Operation zu gewährleisten, ist es notwendig diese Parameter mit entsprechend hoher Genauigkeit zu bestimmen, um danach eine geeignete Ersatzlinse anhand der ermittelten Messwerte auswählen zu können. Die wichtigsten, zu ermittelnden Parameter sind u. a. die Achslänge (Abstand von der Kornea bis zur Retina), die Hornhautkrümmung und -brechkraft, sowie die Länge der Vorderkammer (Abstand von der Kornea bis zur Augenlinse).

So ist es zur Durchführung biometrischer Messungen am Auge von Vorteil, wenn die optische Achse der ophthalmologischen Messanordnung und die optische Achse des zu vermessenden Auges zueinander ausgerichtet sind und idealer weise zusammenfallen. Dadurch kann bei biometrischen Messungen nach dem Prinzip der Kurzkohärenzinterferometrie sichergestellt werden, dass von den schwachen, von den Grenzflächen der Kornea und Linse reflektierten Lichtanteilen eine ausreichende Signalintensität auf den Detektor gelangt und einen messbaren Interferenzkontrast erzeugt.

Nach dem bekannten Stand der Technik sind verschiedene Lösungen zur interferometrischen Bestimmung von Abständen im Auge bekannt, die jeweils unterschiedlich hohe Anforderungen an die Ausrichtung des Auges auf das ophthalmologische Gerät stellen.

Eine erste Anordnung zur interferometrischen Messung der Abstände der vorderen Augenabschnitte wird von Drexler, W. und anderen in [1] beschrieben.
Zur Ausrichtung der Sehachse des Auges auf die optische Achse der Messanordnung wird ein kollimiertes Fixierlicht entlang einer festen, koaxialen Richtung in den Mess-Strahlengang eingespiegelt. Die Einstellung des Winkels zwischen der Sehachse des Patienten und der optischen Achse der Messanordnung erfolgt mit Hilfe eines Scanspiegels. Dabei muss die Ausrichtung der beiden Achsen mit einer Genauigkeit von besser als 1° erfolgen, da andernfalls keine Überlappung von Kornea- und Linsenreflex auf dem Detektor ergibt und kein auswertbares Interferenzsignal entsteht. Entsprechend hoch ist die Empfindlichkeit des Aufbaus gegenüber Verkippungen des Patientenauges. Die Lage der Sehachse des Auges wird hierbei mittels Scannen über einen vorbestimmten Winkelbereich in zwei orthogonalen Raumrichtungen bestimmt. Die hier beschriebene Methode ist sehr zeitaufwendig und zudem für den klinischen Alltag nicht zuverlässig genug.

Eine weitere Lösung zur Ermittlung von Abständen am vorderen Augenabschnitt, vorzugsweise des Pupillen- und/oder Irisdurchmessers wird in der DE 101 08 797 A1 beschrieben. Auch hier wird dem Probanden eine Lichtmarke angeboten auf die er sein Auge fixiert. Der Bediener kann sich während der gesamten Zeit der Einjustierung und der Vermessung visuell davon überzeugen, dass der Proband richtig fixiert. Im Gegensatz zum schematischen Auge nach Gullstrand können bei realen Augen Sehachse und optische Achse bis zu 8° voneinander abweichen, da die Fovea von 3° nasal bis 8° temporal versetzt liegen kann. Entsprechend kann es hierbei vorteilhaft sein, dem Probanden ein versetztes Fixierlicht anzubieten. Wie auch in der zuvor beschriebenen Lösung muss die Ausrichtung beiden Achsen zueinander mit einer hohen Genauigkeit erfolgen. Dies ist hier von besonderer Wichtigkeit, da bei der Bestimmung von Pupillen- und/oder Irisdurchmesser nicht Streu- sondern Reflexlicht detektiert wird.

Eine deutlich verbesserte Methode wird in US 7,380,939 beschrieben. Hier wird anstelle eines kostenintensiven Scanspiegels ein von einem LC-Display erzeugtes Fixierlicht eingesetzt. Dieses ist nicht nur in seiner lateralen Position, sondern auch in seiner scheinbaren Entfernung vom Patientenauge variabel einstellbar ist, um einer eventuellen Fehlsichtigkeit des Patienten Rechnung zu tragen.

Zudem werden durch absichtliche Defokussierung des Messstrahls mittels axialer Verschiebung der Fokussierlinse relativ zum Patientenauge Kornea- und Linsenreflex unscharf und vergrößert auf dem Detektor abgebildet. Dadurch wird die Empfindlichkeit der Messanordnung gegenüber Verkippung oder seitlicher Verschiebung des Patientenauges etwas abgemildert.

Nachteilig ist jedoch, dass aufgrund der Verwendung eines dual-beam Interferometers die von verschiedenen Grenzflächen im Auge reflektierten Messlichtanteile das Interferenzsignal erzeugen, also kein externes Referenzlicht zur Erzeugung des Interferenzkontrastes dient. Aus diesem Grund kann nur eine nicht beugungsbegrenzte Abbildung des Reflexlichtes auf den Photodetektor gewählt werden, sodass nur spekulare (Fresnel-)Reflexe zum Interferenzsignal beitragen. Die von Volumenstreulicht verursachten Speckle sind in der Detektorebene wesentlich kleiner als die Detektorfläche und werden daher über der Detektorfläche "herausgemittelt". Durch diesen Umstand bleibt das Messverfahren weiterhin auf eine im Allgemeinen sehr präzise Vorjustierung auf die Sehachse des Patientenauges angewiesen.

Eine dritte, aus den Schriften WO 01/38820 A1 und WO 2007/053971 A1 bekannte, technische Lösung verwendet zwar eine beugungsbegrenzte Erfassung des im Auge zurück gestreuten Messlichtes zur interferometrischen Bestimmung von Abständen im Auge, verfügt aber nur über ein auf der optischen Achse der Messanordnung fest positioniertes Fixierlicht. Mit dieser Anordnung kann also keine für die Messung vorteilhafte variable Fixierung der Blickrichtung erreicht werden.

### Literatur:

[1] Drexler, W. und andere; "Submicrometer Precision Biometry of the anterior Segment of the Human Eye"; Investigative Ophthalmology & Visual Science, Vol. 38, Nr. 7, Seite 1304ff

Erkenntnisse aus der Praxis haben überraschender Weise gezeigt, dass es in der Nähe des sogenannten Glanzwinkels zu einer erhöhten Intensität der Rückstreuung kommt. Obwohl bei einer beugungsbegrenzten Detektion das erzeugte Volumenstreulicht ausgenutzt wird, das im Wesentlichen in alle Richtungen gleichmäßig remittiert, würde somit eine zumindest grobe Vorjustage der optischen Achse des ophthalmologischen Messgerätes bezüglich der Sehachse zu deutlich verbesserten Messergebnissen führen.

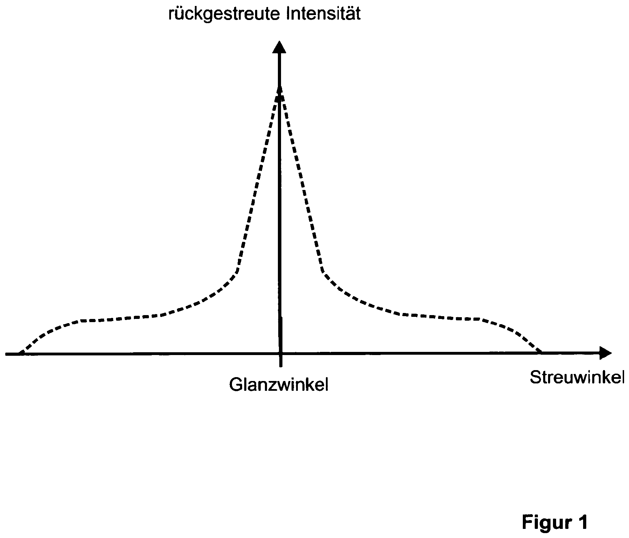

Hierzu zeigt die **Figur 1** den Intensitätsverlauf der von einer Grenzfläche im Auge zurück gestreuten Lichtanteile in Abhängigkeit des Streuwinkels. Es ist zu sehen, dass die zurück gestreuten Lichtanteile in einem schmalen Bereich um den sogenannten Glanzwinkel eine wesentlich höhere Intensität aufweisen als im restlichen Bereich.

Der vorliegenden Erfindung liegt deshalb die Aufgabe zugrunde, eine Lösung zur interferometrischen Bestimmung verschiedener biometrischer Parameter eines Auges zu entwickeln, welche genaue und zuverlässige Messwerte liefert und dennoch nicht auf eine hochgenaue Ausrichtung des Gerätes auf das Auge eines Patienten angewiesen ist. Dabei soll eine hinreichend gute Ausrichtung der optischen Achse einer interferometrischen Messanordnung bezüglich der optischen Achse eines Auges vorzugsweise automatisch erfolgen, wobei sowohl Verkippungen oder seitlicher Verschiebungen des Auges des Patienten als auch dessen Fehlsichtigkeit bei der Ausrichtung berücksichtigt werden sollen.

Erfindungsgemäß wird die Aufgabe durch die Merkmale der unabhängigen Ansprüche gelöst. Bevorzugte Weiterbildungen und Ausgestaltungen sind Gegenstand der abhängigen Ansprüche.

Die Aufgabe wird mit dem erfindungsgemäßen Verfahren zur interferometrischen Bestimmung verschiedener biometrischer Parameter eines Auges, bei dem das Auge von einer Messlichtquelle mit einem Messstrahl und von einer Fixierlichtquelle mit einer Fixiermarke beleuchtet, von einem Bildsensor mit den entstandenen Reflexen aufgenommen und aus der Position der Reflexe in Bezug auf den Mittelpunkt der Pupille oder Iris von einer Auswerteeinheit die Winkelabweichung der optischen Achse des Auges von der optischen Achse des biometrischen Messsystems bestimmt wird, dadurch gelöst, dass vor der Bestimmung der verschiedenen biometrischen Parameter die Ausrichtung des biometrischen Messsystems erfolgt, indem
A) das Auge zur Umfixierung von der, auf Basis der errechneten Winkelabweichung von der Fixierlichtquelle mit einer lateral verschobenen Fixiermarke beleuchtet,
B) das Auge vom Bildsensor mit den entstandenen Reflexen aufgenommen,
C) aus der Position der Reflexe in Bezug auf den Mittelpunkt der Pupille oder Iris von der Auswerteeinheit die Winkelabweichung der optischen Achse des Auges von der optischen Achse des ophthalmologischen Gerätes bestimmt,
D) die ermittelte Winkelabweichung von der Auswerteeinheit mit einer vorgegebenen Toleranz verglichen und
E) die Verfahrensschritte A) bis D) wiederholt werden, wenn die ermittelte Winkelabweichung eine vorgegebene Toleranz übersteigt.

Anderenfalls erfolgt die Bestimmung verschiedener biometrischer Parameter eines Auges, indem
F) das vom Auge reflektierte Messlicht in Form von quasi beugungsbegrenztem Volumenstreulicht detektiert,
G) als Messstrahl mit einem externen Referenzstrahl überlagert, auf den Messsensor geleitet und
H) die interferometrischen Messsignale von der Auswerteeinheit ausgewertet werden.

Mit der erfindungsgemäßen Vorrichtung zur interferometrischen Bestimmung verschiedener biometrischer Parameter eines Auges, bestehend aus einer interferometrischen Messanordnung, einer Messlichtquelle zur Beleuchtung eines Auges mit Messlicht, einer Fixierlichtquelle zur Beleuchtung des Auges mit einer Fixiermarke, einem Bildsensor zur Aufnahme des Auges mit den entstandenen Reflexen und einer Auswerteeinheit zur Bestimmung der Winkelabweichung der optischen Achse des Auges von der optischen Achse des biometrischen Messsystems, wird die Aufgabe dadurch gelöst, dass die Fixierlichtquelle so ausgebildet ist, dass die zu erzeugende Fixiermarke lateral verschiebbar ist, eine vorhandene Optik so ausgebildet ist, dass in erster Linie quasi beugungsbegrenztes Volumenstreulicht detektiert wird, die interferometrische Messanordnung zur Überlagerung des Messlichtes mit externem Referenzlicht ausgebildet ist und die Auswerteeinheit in der Lage ist, die ermittelte Winkelabweichung mit einer vorgegebenen Toleranz zu vergleichen und in dessen Ergebnis zur Umfixierung von der Fixierlichtquelle eine, auf Basis der errechneten Winkelabweichung lateral verschobene Fixiermarke erzeugen lässt oder die biometrische Vermessung veranlasst.

Obwohl die vorgeschlagene technische Lösung vorrangig für ophthalmologische Geräte zur biometrischen Messung der Abstände und Radien im Auge vorgesehen ist, kann sie prinzipiell für andere Geräte in der Ophthalmologie verwendet werden.

Die Erfindung wird nachfolgend anhand von Ausführungsbeispielen näher beschrieben. Dazu zeigen:
- Figur 1:: die Intensität der von einer Grenzfläche im Auge zurück gestreuten Lichtanteile in Abhängigkeit des Streuwinkels,
- Figur 2:: ein Flussdiagramm des erfindungsgemäßen Verfahrens und
- Figur 3:: eine Prinzipdarstellung der erfindungsgemäßen Vorrichtung.

Bei dem erfindungsgemäßen Verfahren zur interferometrischen Bestimmung verschiedener biometrischer Parameter eines Auges wird das Auge von einer Messlichtquelle mit einem Messstrahl und von einer Fixierlichtquelle mit einer Fixiermarke beleuchtet, von einem Bildsensor mit den entstandenen Reflexen aufgenommen und aus der Position der Reflexe in Bezug auf den Mittelpunkt der Pupille oder Iris von einer Auswerteeinheit die Winkelabweichung der optischen Achse des Auges von der optischen Achse des biometrischen Messsystems bestimmt. Während die Fixierlichtquelle zwangsläufig Licht im sichtbaren Spektralbereich aussenden muss, strahlt die Messlichtquelle einen Messstrahl in einem für den Patienten nicht sichtbaren Spektralbereich, was die Auswertung der aufgenommenen Bilder wesentlich vereinfacht.

Dabei erfolgt vor der Bestimmung der verschiedenen biometrischen Parameter die Ausrichtung des biometrischen Messsystems, indem das Auge in einem ersten Verfahrensschritt A) zur Umfixierung von der, auf Basis der errechneten Winkelabweichung von der Fixierlichtquelle mit einer lateral verschobenen Fixiermarke beleuchtet und im nächsten Verfahrensschritt B) vom Bildsensor mit den entstandenen Reflexen aufgenommen wird.

In einem weiteren Verfahrensschritt C) wird aus der Position der Reflexe in Bezug auf den Mittelpunkt der Pupille oder Iris von der Auswerteeinheit die Winkelabweichung der optischen Achse des Auges von der optischen Achse des biometrischen Messsystems bestimmt und gemäß Verfahrensschritt D) von der Auswerteeinheit mit einer vorgegebenen Toleranz verglichen. Im Ergebnis dieses Vergleiches wird im Verfahrensschritt E) die Wiederholung der Verfahrensschritte A) bis D) veranlasst, wenn die ermittelte Winkelabweichung eine vorgegebene Toleranz übersteigt. Hierbei erfolgt die Angleichung der optischen Achse des Auges an die optische Achse des biometrischen Messsystems indem die Sehachse des Auges durch eine korrigierte Fixiermarke gezielt verändert wird.

Anderenfalls erfolgt die Bestimmung verschiedener biometrischer Parameter eines Auges, indem in einem weiteren Verfahrensschritt F) das vom Auge reflektierte Messlicht in Form von quasi beugungsbegrenztem Volumenstreulicht detektiert, im Verfahrensschritt G) als Messstrahl mit einem externen Referenzstrahl überlagert auf den Messsensor geleitet und im letzten Verfahrensschritt H) die interferometrischen Messsignale von der Auswerteeinheit ausgewertet werden.

Dadurch wird das biometrische Messsystem vor der eigentlichen interferometrischen Messung, zur Bestimmung der Lage von optischen Grenzflächen im Auge auf die Physiologie des Patienten optimiert. Dies erfolgt vorzugsweise automatisch durch die vorgeschlagene Lösung, bei der die Berechnung und Aktivierung der von der Fixierlichtquelle lateral verschobenen Fixiermarke in der beschriebenen Art und Weise automatisch und ohne Zutun des Bedieners erfolgt.

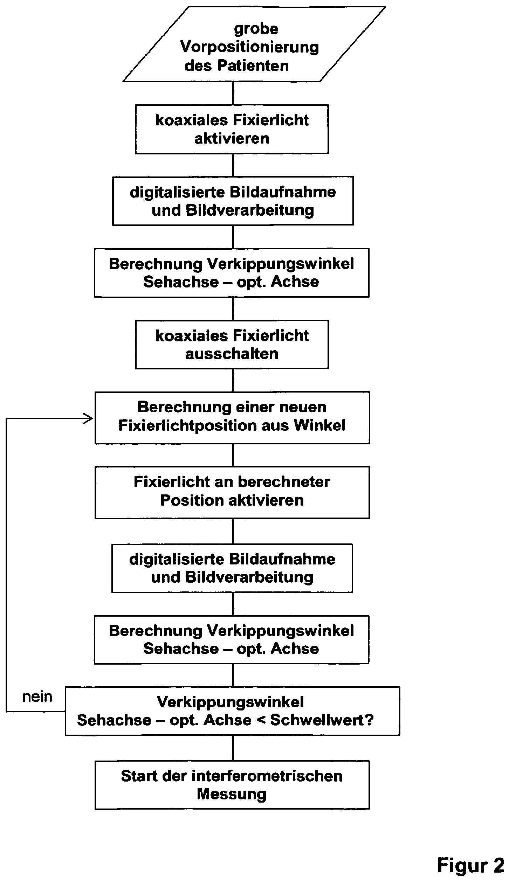

Die Verfahrensschritte zur automatischen Ausrichtung werden dabei so oft durchlaufen, bis die ermittelte Winkelabweichung innerhalb der vorgegebenen Toleranz liegt. Dazu ist in der **Figur 2** ein Flussdiagramm des erfindungsgemäßen Verfahrens dargestellt.

Nach *einer* ,*groben Vorpositionierung des Patienten' wird die ,koaxiale Fixiermarke aktiviert,* auf das Auge abgebildet, zusammen mit dem Auge als *'digitales Bild aufgenommen und ausgewertet und ,der Verkippungswinkel zwischen Sehachse und optischer Achse berechnet'.* Danach wird *die ,koaxiale Fixiermarke deaktiviert*', auf die der Patient die ganze Zeit sein Auge fixiert hatte. Aus dem zuvor berechneten Verkippungswinkel wird nunmehr die Position einer *,korrigierten Fixiermarke berechnet* und die *'Fixiermarke an berechneter Position aktiviert.* Auf diese korrigierte Fixiermarke hat der Patient erneut sein Auge zu fixieren. Erneut wird die Fixiermarke mit dem Auge als *'digitales Bild aufgenommen und ausgewertet'* und *'der Verkippungswinkel zwischen Sehachse und optischer Achse berechnet.* Danach wird überprüft, ob der berechnete *,Verkippungswinkel kleiner als eine vorgegebene Toleranz'* ist. Wenn dies nicht der Fall ist (NEIN), so wird aus dem berechneten Verkippungswinkel erneut die Position einer *'korrigierten Fixiermarke berechnet'* und *die 'Fixiermarke an berechneter Position aktiviert',* ein *'digitales Bild aufgenommen und ausgewertet* usw..

Wenn dies aber der Fall ist (JA), d. h. der berechnete Verkippungswinkel ist kleiner als die vorgegebene Toleranz, so kann die *'biometrische Vermessung starten'.*

Wird hierbei die vorgegebene Toleranz entsprechend klein gewählt, kann gewährleistet werden, dass die optische Achse des biometrischen Messsystems mit der optischen Achse eines Auges zumindest annähernd zusammenfällt. Im Idealfall, bei dem die beiden Achsen tatsächlich zusammenfallen, würde das Verfahren optimale Messsignale liefern.

Die auf der Kurzkohärenzinterferometrie basierende biometrische Vermessung erfolgt hierbei vorzugsweise derart, dass das Messlicht zum einen zumindest annähernd beugungsbegrenzt erfasst und zum anderen mit externem Referenzlicht überlagert wird.

Mit einem auf diese Weise, automatisch ausgerichteten biometrischen Messsystem kann sowohl eine erhöhte Sensitivität erreicht, als auch eine größere Robustheit und Zuverlässigkeit sichergestellt werden.

In einer ersten vorteilhaften Ausgestaltung des Verfahrens wird das Auge von der Fixierlichtquelle mit einer Fixiermarke im sichtbaren Spektralbereich beleuchtet. Der Bildsensor zur Auswertung des Reflexbildes kann entweder den Reflex des Fixierlichtes oder auch den Reflex einer zusätzlichen IR-Lichtquelle detektieren, die koaxial oder beispielsweise ringförmig um die optische Achse des Gerätes herum angeordnet sein kann. Prinzipiell kann auch das Reflexbild der Messlichtquelle zur Bestimmung der Winkelabweichung von Sehachse und optischer Achse des Auges herangezogen werden.

Die Fehlsichtigkeit des zu vermessenden Patientenauges führt dazu, dass die Fixiermarke nicht scharf auf den Augenhintergrund angebildet wird und der Patient unter Umständen Probleme bei deren Fixierung hat. Deshalb sieht eine zweite Ausgestaltung des Verfahrens vor, dass das Auge zum Ausgleich der Fehlsichtigkeit von der Fixierlichtquelle mit einer axial verschiebbaren Fixiermarke beleuchtet wird. Der erforderliche Betrag der axialen Verschiebung kann dabei der Patientenakte entnommen und am biometrischen Messsystem eingestellt werden.

Eine dritte vorteilhafte Ausgestaltung des Verfahrens berücksichtigt den Umstand, dass der überwiegende Teil der Patienten einen vom "idealen Auge" abweichenden Augenaufbau aufweisen. Neben verkürzten oder verlängerten Augen, oder einer verformten Hornhaut, Linse und/oder Retina weist ein Großteil der Patientenaugen eine Verkippung der Linse auf, die insbesondere bei der Augenvermessung hinderlich ist. Deshalb sieht das erfindungsgemäße Verfahren vor, das Auge bereits zu Beginn der Ausrichtung von der Fixierlichtquelle mit einer geringfügig lateral verschobenen Fixiermarke zu beleuchten. Dies hat den Vorteil, dass die automatische Ausrichtung durch den "Wegfall" der Beleuchtung mit einer koaxialen Fixiermarke beschleunigt wird. Hierbei liegt die durchschnittliche Verkippung der Augenlinse bei 2°-5°.

Um die Zuverlässigkeit der aus den vom Auge reflektierten Messstrahlen gewonnenen Messwerte noch einmal zu erhöhen, ist es vorteilhaft die Verfahrensschritte A) bis D) noch einmal zu wiederholen, auch wenn die ermittelte Winkelabweichung die vorgegebene Toleranz nicht übersteigt.

Bei der eigentlichen Bestimmung der verschiedenen biometrischen Parameter, d. h. nach der erfolgten Ausrichtung des biometrischen Messsystems, ist es vorteilhaft, dass das Auge von der Messlichtquelle mit einem kurzkohärenten Messstrahl beleuchtet wird. Die Verwendung kohärenter Strahlung, die hinsichtlich ihrer räumlichen und zeitlichen Ausbreitung eine feste Phasenbeziehung hat, hat sich in der Ophthalmologie insbesondere bei der interferometrischen Entfernungsmessung reflektierender Materialien durchgesetzt. Bei diesem, als optische Kohärenztomografie (engl.: optical coherence tomography, kurz: OCT) bezeichneten Verfahren, wird Licht geringer Kohärenzlänge mit Hilfe eines Interferometers zur Entfernungsmessung genutzt. Die Vorteile gegenüber konkurrierenden Verfahren liegen in der relativ hohen Eindringtiefe in streuendes Gewebe, einer hohen Messgeschwindigkeit und dies bei einer hohen axialen Auflösung.

In der erfindungsgemäßen Ausgestaltung des Verfahrens wird die Apertur für die Detektion des Volumenstreulichtes entsprechend eingegrenzt. Diese zumindest annähernde, beugungsbegrenzte Erfassung hat den Vorteil, dass das aus dem, im Wesentlichen in alle Richtungen gleichmäßig remittierenden Volumenstreulicht gewonnenen Messlicht eine hohe Raumselektivität aufweist.

Eine letzte Ausgestaltung des Verfahrens sieht vor, dass das beugungsbegrenzt erfasste Messlicht mit externem Referenzlicht überlagert wird. Dazu wird der Referenzstrahl des Interferometers vorher ausgekoppelt und nicht mit in das Auge geleitet. Dadurch ist das biometrische Messsystem zwar empfindlich gegen mögliche Bewegungen des Patientenauges, zur Überlagerung mit dem Referenzstrahl kommen allerdings nur Lichtanteile, die auch tatsächlich die Tiefeninformationen beinhalten.

Das erfindungsgemäße Verfahren zeichnet sich dadurch aus, dass eine variable Fixiermarke Verwendung findet, deren laterale Position von der optischen Achse der Messanordnung variabel einstellbar ist und dass das (annähernd) beugungsbegrenzt erfasste Messlicht mit externem Referenzlicht überlagert wird.

Die erfindungsgemäße Vorrichtung zur interferometrischen Bestimmung verschiedener biometrischer Parameter eines Auges besteht aus einer interferometrischen Messanordnung, einer Messlichtquelle zur Beleuchtung eines Auges mit Messlicht, einer Fixierlichtquelle zur Beleuchtung des Auges mit einer Fixiermarke, einem Bildsensor zur Aufnahme des Auges mit den entstandenen Reflexen und einer Auswerteeinheit zur Bestimmung der Winkelabweichung der optischen Achse des Auges von der optischen Achse des biometrischen Messsystems.

Dabei ist die Fixierlichtquelle so ausgebildet, das die zu erzeugende Fixiermarke lateral verschiebbar ist. Das von einer vorhandenen Optik in erster Linie quasi beugungsbegrenzt detektierte Volumenstreulicht wird als Messlicht in der interferometrischen Messanordnung mit externem Referenzlicht überlagert und auf einen vorhandenen Messsensor abgebildet. Die Auswerteeinheit ist in der Lage, die ermittelte Winkelabweichung mit einer vorgegebenen Toleranz zu vergleichen und in dessen Ergebnis zur Umfixierung von der Fixierlichtquelle eine, auf Basis der errechneten Winkelabweichung lateral verschobene Fixiermarke erzeugen zu lassen oder die biometrische Vermessung zu veranlassen.

Während die Fixierlichtquelle zwangsläufig Licht im sichtbaren Spektralbereich aussenden muss, strahlt die Messlichtquelle einen Messstrahl in einem für den Patienten nicht sichtbaren Spektralbereich, was die Auswertung der aufgenommenen Bilder wesentlich vereinfacht.

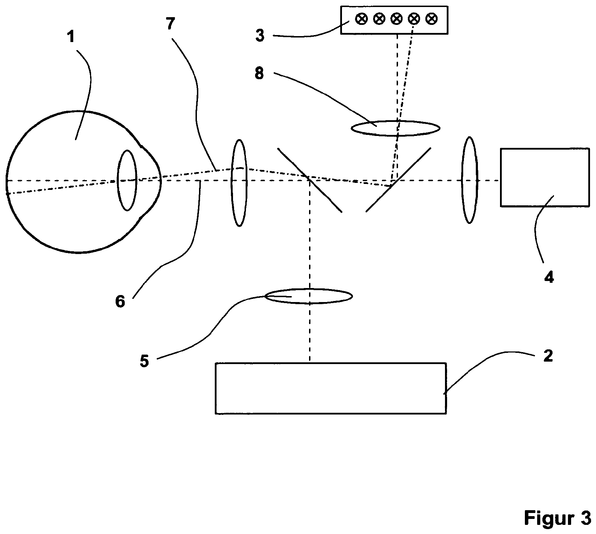

Hierzu zeigt die **Figur 3** eine Prinzipdarstellung der erfindungsgemäßen Vorrichtung zur Bestimmung verschiedener biometrischer Parameter eines Auges **1**. Die Vorrichtung besteht aus einer interferometrischen Messanordnung mit Messlichtquelle und Messsensor **2**, einer Fixierlichtquelle **3** mit einer Abbildungsoptik **8**, einem Bildsensor **4**, einer Optik **5** zur quasi beugungsbegrenzten Detektion von Volumenstreulicht und einer (nicht dargestellten) Auswerteeinheit. Die Darstellung zeigt den ausgerichteten Zustand, bei dem die optische Achse **6** des Auges und des biometrischen Messsystems identisch ist, während die durch das Fixierlicht gekennzeichnete Sehachse **7** des Auges **1** davon abweicht.

Dabei ist die Fixierlichtquelle so ausgebildet, das die zu erzeugende Fixiermarke lateral verschiebbar ist. Weiterhin ist eine Einheit zur Erzeugung einer verringerten Detektionsapertur vorhanden und die interferometrische Messanordnung zur Überlagerung des Messlichtes mit externem Referenzlicht ausgebildet. Die Auswerteeinheit ist außerdem in der Lage, die ermittelte Winkelabweichung mit einer vorgegebenen Toleranz zu vergleichen und in dessen Ergebnis zur Umfixierung von der Fixierlichtquelle eine, auf Basis der errechneten Winkelabweichung lateral verschobene Fixiermarke erzeugen lässt oder die biometrische Vermessung veranlasst.

Hierbei erfolgt die Bestimmung der verschiedenen biometrischen Parameter erst nach Ausrichtung des biometrischen Messsystems. Im einzelnen wird dazu zur Umfixierung von der Fixierlichtquelle auf Basis der von der Auswerteeinheit errechneten Winkelabweichung eine lateral verschobene Fixiermarke erzeugt, auf das Auge abgebildet und vom Bildsensor mit den entstandenen Reflexen aufgenommen.

Die Auswerteeinheit ist in der Lage, aus der Position der Reflexe in Bezug auf den Mittelpunkt der Pupille oder Iris die Winkelabweichung der Sehachse von der optischen Achse des biometrischen Messsystems zu bestimmen und mit einer vorgegebenen Toleranz zu vergleichen. Im Ergebnis dieses Vergleiches wird von der Auswerteeinheit für den Fall, dass die ermittelte Winkelabweichung eine vorgegebene Toleranz übersteigt, die Fixierlichtquelle veranlasst, auf Basis der errechneten Winkelabweichung eine lateral verschobene Fixiermarke zu erzeugen. Hierbei erfolgt die Angleichung der optischen Achse des Auges an die optische Achse des biometrischen Messsystems indem die Sehachse des Auges durch eine korrigierte Fixiermarke gezielt verändert wird.

Für den Fall, dass die ermittelte Winkelabweichung eine vorgegebene Toleranz nicht übersteigt, wird von der Auswerteeinheit die Bestimmung verschiedener biometrischer Parameter eines Auges veranlasst.

Dabei wird von der Einheit zur Erzeugung einer verringerten Detektionsapertur gewährleistet, dass das vom Auge reflektierte Messlicht in Form von Volumenstreulicht beugungsbegrenzt detektiert, in der interferometrischen Messanordnung mit externem Referenzlicht überlagert und auf den Messsensor abgebildet werden kann.

Von der Auswerteeinheit werden die so erzeugten interferometrischen Messsignale ausgewertet und verschiedene biometrische Parameter des Auges berechnet.

Wird hierbei die vorgegebene Toleranz entsprechend klein gewählt, kann gewährleistet werden, dass die optische Achse des biometrischen Messsystems mit der optischen Achse eines Auges nahezu zusammenfällt und annähernd optimale Messsignale erzeugt werden.

Die eigentlichen interferometrischen Messungen, zur Bestimmung der Lage von optischen Grenzflächen im Auge erfolgen somit erst dann, wenn das biometrische Messsystem auf die Physiologie des Patienten eingestellt ist. Dies erfolgt vorzugsweise automatisch durch die vorgeschlagene Lösung, bei der die Berechnung und Aktivierung der von der Fixierlichtquelle lateral verschobenen Fixiermarke in der beschriebenen Art und Weise automatisch und ohne Zutun des Bedieners erfolgt.

Die ersten vorteilhaften Ausgestaltungen der Vorrichtung beziehen sich auf die verwendete Fixierlichtquelle. Vorzugsweise wird eine Fixierlichtquelle verwendet, von der Fixiermarken im sichtbaren Spektralbereich erzeugt werden. Weiterhin bietet sich an, dass die Fixierlichtquelle als LC-Display, ein Array von Lichtquellen oder lateral verschiebbare Einzellichtquelle ausgebildet ist. Es ist aber auch möglich die Fixierlichtquelle koaxial, beispielsweise ringförmig um die optische Achse des Gerätes herum anzuordnen.

Zum anderen führt Fehlsichtigkeit des zu vermessenden Patientenauges dazu, dass die Fixiermarke nicht scharf auf den Augenhintergrund angebildet wird und der Patient unter Umständen Probleme bei deren Fixierung hat. Deshalb ist es vorteilhaft, wenn die Fixierlichtquelle zum Ausgleich der Fehlsichtigkeit über eine Vorrichtung zur Abbildung einer axial verschobenen Fixiermarke verfügt. Der erforderliche Betrag der axialen Verschiebung kann dabei der Patientenakte entnommen und am biometrischen Messsystem eingestellt werden.

Als Vorrichtung zur Abbildung einer axial verschobenen Fixiermarke kann beispielsweise eine Abbildungsoptik in Form eines Zoomobjektives Verwendung finden. Prinzipiell ist es aber auch möglich, dass die Vorrichtung zur Abbildung einer axial verschobenen Fixiermarke, ein Stellantrieb zur axialen Verschiebung der Fixierlichtquelle oder einer Abbildungsoptik ist.

Ausgehend von dem Umstand, dass der überwiegende Teil der Patienten einen vom "idealen Auge" abweichenden Augenaufbau aufweisen, wobei neben verkürzten oder verlängerten Augen, verformten Hornhaut, Linse und/oder Retina oder aber Verkippungen der Linse anzutreffen sind, lässt sich mit der vorgeschlagenen Vorrichtung die Ausrichtung der optischen Achse des biometrischen Messsystems auf die optischen Achse des Auges beschleunigen.

Dazu wird die Fixierlichtquelle veranlasst bereits zu Beginn der Ausrichtung eine geringfügig lateral verschobene Fixiermarke zu erzeugen und auf das Auge abzubilden. Dies hat den Vorteil, dass die automatische Ausrichtung durch den "Wegfall" der Beleuchtung mit einer koaxialen Fixiermarke beschleunigt wird. Hierbei liegt die durchschnittliche Verkippung der Augenlinse bei 2°-5°.

Die zweiten vorteilhaften Ausgestaltungen der Vorrichtung beziehen sich auf die verwendete Messlichtquelle. Vorzugsweise ist die Messlichtquelle eine, einen kurzkohärenten Messstrahl aussendende Messlichtquelle. Die Verwendung kohärenter Strahlung, die hinsichtlich ihrer räumlichen und zeitlichen Ausbreitung eine feste Phasenbeziehung hat, hat sich in der Ophthalmologie insbesondere bei der interferometrischen Entfernungsmessung reflektierender Materialien durchgesetzt. Bei diesem, als optische Kohärenztomografie (engl.: optical coherence tomography, kurz: OCT) bezeichneten Verfahren, wird Licht geringer Kohärenzlänge mit Hilfe eines Interferometers zur Entfernungsmessung genutzt. Die Vorteile gegenüber konkurrierenden Verfahren liegen in der relativ hohen Eindringtiefe in streuendes Gewebe, eine hoher Messgeschwindigkeit und dies bei einer hohen axialen Auflösung.

Die zweiten vorteilhaften Ausgestaltungen der Vorrichtung beziehen sich auf die verwendete Einheit zur Erzeugung einer verringerten Detektionsapertur. Die Einheit ist beispielsweise eine Blende oder eine Single-Mode-Faser. Diese zumindest annähernde, beugungsbegrenzte Erfassung hat den Vorteil, dass das aus dem, im Wesentlichen in alle Richtungen gleichmäßig remittierenden Volumenstreulicht gewonnenen Messlicht eine hohe Raumselektivität aufweist.

Das beugungsbegrenzt erfasste Messlicht wird in der interferometrischen Messanordnung mit externem Referenzlicht überlagert. Dazu wird der Referenzstrahl des Interferometers vorher ausgekoppelt und nicht mit in das Auge geleitet. Dadurch ist das biometrische Messsystem zwar empfindlich gegen mögliche Bewegungen des Patientenauges, zur Überlagerung mit dem Referenzstrahl kommen allerdings nur Lichtanteile, die auch tatsächlich die Tiefeninformationen beinhalten.

Die erfindungsgemäße Vorrichtung zeichnet sich durch die Kombination einer Fixierlichtquelle, zur Erzeugung einer lateral verschiebbaren Fixiermarke, einer Einheit zur Erzeugung einer verringerten Detektionsapertur, einer interferometrischen Messanordnung zur Überlagerung des Messlichtes mit externem Referenzlicht und eine Auswerteeinheit aus, die in der Lage ist, die ermittelte Winkelabweichung mit einer vorgegebenen Toleranz zu vergleichen und in dessen Ergebnis zur Umfixierung von der Fixierlichtquelle eine, auf Basis der errechneten Winkelabweichung die Erzeugung einer lateral verschobenen Fixiermarke oder die biometrische Vermessung zu veranlassen.

Mit dem erfindungsgemäßen Verfahren und der dafür geeigneten Vorrichtung wird eine Lösung zur interferometrischen Bestimmung verschiedener biometrischer Parameter eines Auges zur Verfügung gestellt, welche genaue und zuverlässige Messwerte liefert und dennoch nicht auf eine hochgenaue Ausrichtung des Gerätes auf das Auge eines Patienten angewiesen ist. Dabei erfolgt die hinreichend gute Ausrichtung der optischen Achse einer interferometrischen Messanordnung bezüglich der optischen Achse eines Auges vorzugsweise automatisch, wobei sowohl Verkippungen oder seitlicher Verschiebungen des Auges des Patienten als auch dessen Fehlsichtigkeit bei der Ausrichtung berücksichtigt werden können.

Die vorliegende Erfindung betrifft eine Lösung zur interferometrischen Bestimmung verschiedener biometrischer Parameter eines Auges, bei der die optische Achse des biometrischen Messsystems zur optischen Achse des Auges eines Auges ausgerichtet wird und idealer weise mit dieser zusammenfällt.

Die auf der Kurzkohärenzinterferometrie basierende biometrische Vermessung erfolgt hierbei vorzugsweise derart, dass das Messlicht zum einen zumindest annähernd beugungsbegrenzt erfasst und zum anderen mit externem Referenzlicht überlagert wird.

Mit einem auf diese Weise, automatisch ausgerichteten biometrischen Messsystem kann sowohl eine erhöhte Sensitivität erreicht, als auch eine größere Robustheit und Zuverlässigkeit sichergestellt werden.

## Patentansprüche

1. Verfahren zur interferometrischen Bestimmung verschiedener biometrischer Parameter eines Auges, bei dem das Auge von einer Messlichtquelle mit einem Messstrahl und von einer Fixierlichtquelle mit einer Fixiermarke beleuchtet, von einem Bildsensor mit den entstandenen Reflexen aufgenommen und aus der Position der Reflexe in Bezug auf den Mittelpunkt der Pupille oder Iris von einer Auswerteeinheit die Winkelabweichung der optischen Achse des Auges von der optischen Achse des biometrischen Messsystems bestimmt wird, wobei vor der Bestimmung der verschiedenen biometrischen Parameter die Ausrichtung des biometrischen Messsystems erfolgt, indem
A) das Auge zur Umfixierung von der, auf Basis der errechneten Winkelabweichung von der Fixierlichtquelle mit einer lateral verschobenen Fixiermarke beleuchtet,
B) das Auge vom Bildsensor mit den entstandenen Reflexen aufgenommen,
C) aus der Position der Reflexe in Bezug auf den Mittelpunkt der Pupille oder Iris von der Auswerteeinheit die Winkelabweichung der optischen Achse des Auges von der optischen Achse des biometrischen Messsystems bestimmt, **dadurch gekennzeichnet, dass** weiterhin
D) die ermittelte Winkelabweichung von der Auswerteeinheit mit einer vorgegebenen Toleranz verglichen und
E) die Verfahrensschritte A) bis D) wiederholt werden, wenn die ermittelte Winkelabweichung eine vorgegebene Toleranz übersteigt und
anderenfalls die Bestimmung verschiedener biometrischer Parameter eines Auges erfolgt, indem
F) das vom Auge reflektierte Messlicht in Form von quasi beugungsbegrenztem Volumenstreulicht detektiert,
G) als Messstrahl mit einem externen Referenzstrahl überlagert, auf einen Messsensor geleitet und
H) die interferometrischen Messsignale von der Auswerteeinheit ausgewertet werden.

2. Verfahren nach Anspruch 1, **dadurch gekennzeichnet, dass** das Auge mit Fixierlicht aus einer sichtbares Licht abstrahlenden Fixierlichtquelle beleuchtet wird.

3. Verfahren nach mindestens einem der Ansprüche 1 und 2, **dadurch gekennzeichnet, dass** das Auge zum Ausgleich seiner Fehlsichtigkeit von der Fixierlichtquelle mit einer axial verschobenen Fixiermarke beleuchtet wird.

4. Verfahren nach mindestens einem der vorgenannten Ansprüche, **dadurch gekennzeichnet, dass** das Auge bereits zu Beginn der Ausrichtung unter Berücksichtigung einer durchschnittlichen Verkippung der Augenlinse von 2-5° von der Fixierlichtquelle mit einer geringfügig lateral verschobenen Fixiermarke beleuchtet wird.

5. Verfahren nach mindestens einem der vorgenannten Ansprüche, **dadurch gekennzeichnet, dass** das Auge mit den entstandenen Reflexen aufgenommen von einem ortsauflösenden Bildsensor aufgenommen wird.

6. Verfahren nach mindestens einem der vorgenannten Ansprüche, **dadurch gekennzeichnet, dass** die Verfahrensschritte A) bis D) zur Erhöhung der Zuverlässigkeit der aus den vom Auge reflektierten Messstrahl gewonnenen Messwerte noch einmal wiederholt werden.

7. Verfahren nach mindestens einem der vorgenannten Ansprüche, **dadurch gekennzeichnet, dass** das Auge von der Messlichtquelle mit einem kurzkohärenten Messstrahl beleuchtet wird.

8. Verfahren nach mindestens einem der vorgenannten Ansprüche, **dadurch gekennzeichnet, dass** von einer Optik in erster Linie Volumenstreulicht detektiert wird.

9. Verfahren nach mindestens einem der vorgenannten Ansprüche, **dadurch gekennzeichnet, dass** der externe Referenzstrahl des Interferometers vorher ausgekoppelt und nicht mit in das Auge geleitet wird.

10. Vorrichtung zur interferometrischen Bestimmung verschiedener biometrischer Parameter eines Auges (1), bestehend aus einer interferometrischen Messanordnung, einer Messlichtquelle zur Beleuchtung des Auges (1) mit Messlicht, einer Fixierlichtquelle (3) zur Beleuchtung des Auges (1) mit einer Fixiermarke, einem Bildsensor (4) zur Aufnahme des Auges (1) mit den entstandenen Reflexen und einer Auswerteeinheit zur Bestimmung der Winkelabweichung der optischen Achse (6) des Auges von der optischen Achse des biometrischen Messsystems, **dadurch gekennzeichnet, dass** die Fixierlichtquelle (3) so ausgebildet ist, das die zu erzeugende Fixiermarke lateral verschiebbar ist, eine vorhandene Optik (5) so ausgebildet ist, dass in erster Linie quasi beugungsbegrenztes Volumenstreulicht detektiert wird, die interferometrische Messanordnung zur Überlagerung des Messlichtes mit externem Referenzlicht ausgebildet ist und über einen Messsensor verfügt und die Auswerteeinheit in der Lage ist, die ermittelte Winkelabweichung mit einer vorgegebenen Toleranz zu vergleichen und in dessen Ergebnis zur Umfixierung von der Fixierlichtquelle (3) eine, auf Basis der errechneten Winkelabweichung lateral verschobene Fixiermarke erzeugen lässt oder die biometrische Vermessung veranlasst.

11. Vorrichtung nach Anspruch 10, **dadurch gekennzeichnet, dass** die eine sichtbare Fixiermarke erzeugende Fixierlichtquelle (3) vorzugsweise ein LC-Display, ein Array von Lichtquellen oder eine lateral verschiebbare Einzellichtquelle ist.

12. Vorrichtung nach mindestens einem der Ansprüche 10 bis 11, **dadurch gekennzeichnet, dass** die Fixierlichtquelle (3) über eine Vorrichtung zur Abbildung einer axial verschobenen Fixiermarke verfügt.

13. Vorrichtung nach Anspruche 12, **dadurch gekennzeichnet, dass** die Vorrichtung zur Abbildung einer axial verschobenen Fixiermarke, eine Abbildungsoptik (8) in Form einer Zoomobjektives oder ein Stellantrieb zur axialen Verschiebung der Fixierlichtquelle (3) oder einer Abbildungsoptik (8) ist.

14. Vorrichtung nach Anspruche 10, **dadurch gekennzeichnet, dass** der Bildsensor (4) zur Aufnahme des Auges (1) mit den entstandenen Reflexen ein ortsauflösender Bildsensor ist.

15. Vorrichtung nach Anspruch 10, **dadurch gekennzeichnet, dass** die Messlichtquelle eine, einen kurzkohärenten Messstrahl aussendende Messlichtquelle ist.

16. Vorrichtung nach Anspruch 10, **dadurch gekennzeichnet, dass** die Optik (5) zur Detektion von quasi beugungsbegrenztem Volumenstreulicht eine Single-Mode-Faser ist.

## Claims

1. Method for interferometric determination of various biometric parameters of an eye, in which the eye is illuminated by a measurement beam from a measurement light source and by a fixation marker from a fixation light source and recorded with the reflections that arose by an image sensor, and in which the angular deviation of the optical axis of the eye from the optical axis of the biometric measurement system is determined by an evaluation unit from the position of the reflections in relation to the centre point of the pupil or iris, wherein, prior to determining the various biometric parameters, the biometric measurement system is aligned by virtue of
A) the eye illuminated by the fixation light source with a laterally displaced fixation marker for the purposes of a re-fixation of the on the basis of the calculated angular deviation,
B) the eye recorded with the reflections that arose by the image sensor,
C) the angle deviation of the optical axis of the eye from the optical axis of the biometric measurement system determined by the evaluation unit from the position of the reflections in relation to the centre point of the pupil or iris, **characterized in that**, furthermore,
D) the established angle deviation is compared to a predetermined tolerance by the evaluation unit and
E) method steps A) to D) are repeated if the established angular deviation exceeds a predetermined tolerance, and,
otherwise, various biometric parameters of an eye are determined by virtue of
F) the measurement light reflected by the eye being detected in the form of virtually diffraction-limited subsurface scattered light,
G) said light being superposed as a measurement beam onto an external reference beam and guided onto a measurement sensor, and
H) the interferometric measurement signals being evaluated by the evaluation unit.

2. Method according to Claim 1, **characterized in that** the eye is illuminated by fixation light from a fixation light source emitting light in the visible spectral range.

3. Method according to Claim 1 and/or Claim 2, **characterized in that**, in order to compensate the refractive error of the eye, the latter is illuminated with an axially displaced fixation marker by the fixation light source

4. Method according to at least one of the preceding claims, **characterized in that** the eye is already illuminated with a slightly laterally displaced fixation marker by the fixation light source at the start of the alignment, taking into account an average tilt of the eye lens of 2-5°.

5. Method according to at least one of the preceding claims, **characterized in that** the eye with the reflections that arose is recorded is recorded by a spatially resolving image sensor.

6. Method according to at least one of the preceding claims, **characterized in that** method steps A) to D) are once again repeated for the purposes of increasing the reliability of the measurement values obtained from the measurement beam reflected by the eye.

7. Method according to at least one of the preceding claims, **characterized in that** the eye is illuminated by the measurement light source with a measurement beam with a short coherence length.

8. Method according to at least one of the preceding claims, **characterized in that** an optical unit primarily detects subsurface scattered light.

9. Method according to at least one of the preceding claims, **characterized in that** the external reference beam of the interferometer is decoupled in advance and not also guided into the eye.

10. Apparatus for interferometric determination of various biometric parameters of an eye (1), consisting of an interferometric measurement arrangement, a measurement light source for illuminating the eye (1) with measurement light, a fixation light source (3) for illuminating the eye (1) with a fixation marker, an image sensor (4) for recording the eye (1) with the reflections that arose, and an evaluation unit for determining the angular deviation of the optical axis (6) of the eye from the optical axis of the biometric measurement system, **characterized in that** the fixation light source (3) is embodied in such a way that the fixation marker to be generated is laterally displaceable, and a present optical unit (5) is embodied in such a way that primarily virtually diffraction-limited subsurface scattered light is detected, the interferometric measurement arrangement is embodied to superpose the measurement light onto external reference light and comprises a measurement sensor, and the evaluation unit is able to compare the established angle deviation to a predetermined tolerance and, as a result thereof, causes a laterally displaced fixation marker to be generated on the basis of the calculated angular deviation for re-fixation of the fixation light source (3) or triggers the biometric measurement.

11. Apparatus according to Claim 10, **characterized in that** the fixation light source (3) generating an fixation marker in the visible spectral range is preferably an LC display, an array of light sources or a laterally displaceable individual light source.

12. Apparatus according to Claim 10 and/or claim 11, **characterized in that** the fixation light source (3) comprises an apparatus for imaging an axially displaced fixation marker.

13. Apparatus according to Claim 12, **characterized in that** the apparatus for imaging an axially displaced fixation marker is an imaging optical unit (8) in the form of a zoom lens or an actuator for axially displacing the fixation light source (3) or an imaging optical unit (8).

14. Apparatus according to Claim 10, **characterized in that** the image sensor (4) for recording the eye (1) with the reflections that arose is a spatially resolving image sensor.

15. Apparatus according to Claim 10, **characterized in that** the measurement light source is a measurement light source emitting a measurement beam with a short coherence length.

16. Apparatus according to Claim 10, **characterized in that** the optical unit (5) for detecting virtually diffraction-limited subsurface scattering light is a single-mode fibre.

## Revendications

1. Procédé pour déterminer des paramètres biométriques différents d'un oeil par interférométrie, dans lequel l'oeil est éclairé par une source de lumière de mesure au moyen d'un faisceau de mesure et par une source de lumière de fixation avec un repère de fixation, l'oeil est acquis par un capteur d'image à partir des réflexions occasionnées et l'écart angulaire de l'axe optique de l'oeil par rapport à l'axe optique du système de mesure biométrique est déterminé à partir de la position des réflexions par rapport au centre de la pupille ou de l'iris par une unité d'évaluation, dans lequel
avant la détermination des différents paramètres biométriques, l'orientation du système biométrique est effectuée par les opérations suivantes :
A) l'oeil éclairé par la source de lumière de fixation avec un repère de fixation décalé latéralement pour la refixation de la sur la base de l'écart angulaire calculé,
B) l'oeil acquis par le capteur d'image au moyen des réflexions occasionnées,
C) l'écart angulaire de l'axe optique de l'oeil par rapport à l'axe optique du système de mesure biométrique déterminé à partir de la position des réflexions par rapport au centre de la pupille ou de l'iris, **caractérisé en ce qu'**en outre :
D) l'écart angulaire déterminé est comparé par l'unité d'évaluation à une tolérance prédéfinie, et
E) les étapes de procédé A) à D) sont répétées lorsque l'écart angulaire déterminé dépasse une tolérance prédéfinie, et
sinon, la détermination de différents paramètres biométriques d'un oeil est effectuée par les opérations suivantes :
F) la lumière de mesure réfléchie par l'oeil est détectée sous la forme d'une lumière diffusée en volume quasiment limitée par la diffraction,
G) elle est superposée en tant que faisceau de mesure à un faisceau de référence externe, et est acheminée à un capteur de mesure, et
H) des signaux de mesure interférométriques sont évalués par l'unité d'évaluation.

2. Procédé selon la revendication 1, **caractérisé en ce que** l'oeil est éclairé par une lumière de fixation provenant d'une source de lumière de fixation émettant de la lumière visible.

3. Procédé selon au moins l'une des revendications 1 et 2, **caractérisé en ce que** l'oeil est éclairé par la source de lumière de fixation avec un repère de fixation décalé axialement pour compenser son défaut de vision.

4. Procédé selon au moins l'une des revendications précédentes, **caractérisé en ce que** l'oeil est éclairé par la source de lumière de fixation avec un repère de fixation légalement décalé latéralement au début de l'orientation en tenant compte d'une inclinaison moyenne du cristallin de 2-5°.

5. Procédé selon au moins l'une des revendications précédentes, **caractérisé en ce que** l'oeil avec des réflexions occasionnées est acquis est acquis par un capteur d'image à résolution spatiale.

6. Procédé selon au moins l'une des revendications précédentes, **caractérisé en ce que** les étapes de procédé A) à D) sont effectuées encore une fois pour augmenter la fiabilité des valeurs de mesure obtenues à partir du faisceau de mesure réfléchi par l'oeil.

7. Procédé selon au moins l'une des revendications précédentes, **caractérisé en ce que** l'oeil est éclairé par la source de lumière de mesure avec un faisceau de mesure à faible longueur de cohérence.

8. Procédé selon au moins l'une des revendications précédentes, **caractérisé en ce que** la lumière diffusée en volume est principalement détectée par une optique.

9. Procédé selon au moins l'une des revendications précédentes, **caractérisé en ce que** le faisceau de référence externe de l'interféromètre est préalablement couplé en sortie et n'est pas guidé vers l'oeil.

10. Dispositif pour déterminer des paramètres biométriques différents d'un oeil (1) par interférométrie, constitué d'un système de mesure interférométrique, d'une source de lumière de mesure destinée à éclairer l'oeil (1) avec une lumière de mesure, d'une source de lumière de fixation (3) destinée à éclairer l'oeil (1) avec un repère de fixation, d'un capteur d'image (4) destiné à acquérir l'oeil (1) au moyen des réflexions occasionnées et d'une unité d'évaluation destinée à déterminer l'écart angulaire de l'axe optique (6) de l'oeil par rapport à l'axe optique du système de mesure biométrique, **caractérisé en ce que** la source de lumière de fixation (3) est réalisée de manière à ce que le repère de fixation à générer puisse être décalé latéralement, **en ce qu'**une optique présente (5) est réalisée de manière à ce qu'une lumière diffusée en volume quasiment limitée par la diffraction soit principalement détectée, **en ce que** le système de mesure interférométrique est réalisé de manière à superposer la lumière de mesure à une lumière de référence externe et comporte un capteur de mesure et **en ce que** l'unité d'évaluation est conçue pour comparer l'écart angulaire déterminé à une tolérance prédéfinie et, en fonction du résultat, pour la fixation d'une source de lumière de fixation (3), provoque la génération d'un repère de fixation décalé latéralement sur la base de l'écart angulaire calculé, ou déclenche la mesure biométrique.

11. Dispositif selon la revendication 10, **caractérisé en ce que** la source de lumière de fixation (3) générant un repère de fixation visible est de préférence un afficheur à cristaux liquides, un réseau de sources de lumière ou une source de lumière individuelle pouvant être décalée latéralement.

12. Dispositif selon au moins l'une des revendications 10 à 11, **caractérisé en ce que** la source de lumière de fixation (3) comporte un dispositif destiné à former l'image d'un repère de fixation décalé axialement pour compenser son défaut de vision.

13. Dispositif selon la revendication 12, **caractérisé en ce que** le dispositif destiné à former l'image d'un repère de fixation décalé axialement est une optique de formation d'image (8) sous la forme d'un objectif à focale variable ou un actionneur permettant le décalage axial de la source de lumière de fixation (3) ou d'une optique de formation d'image (8).

14. Dispositif selon la revendication 10, **caractérisé en ce que** le capteur d'image (4) est un capteur d'image à résolution spatiale destiné à acquérir l'oeil (1) au moyen des réflexions occasionnées.

15. Dispositif selon la revendication 10, **caractérisé en ce que** la source de lumière de mesure est une source de lumière de mesure émettant un faisceau de mesure à faible longueur de cohérence.

16. Dispositif selon la revendication 10, **caractérisé en ce que** l'optique (5) destinée à la diffraction d'une lumière diffusée en volume quasiment limitée par la diffraction est une fibre monomode.
